# EUROPEAN PATENT APPLICATION

(11) **EP 4 292 549 A1**
(43) Date of publication of application: **20.12.2023**
(21) Application number: 22752149.9
(22) Date of filing: 27.01.2022
(51) Int. Cl.: A61B 17/34, A61B 34/30

(54) **GUIDABLE AND DEVELOPABLE INSTRUMENT AND SURGICAL ROBOT SYSTEM**

(30) Priority: 09.02.2021 CN 202110178377
(71) Applicant: Beijing Surgerii Robotics Company Limited, Beijing 100192 (CN)
(72) Inventor: XU, Kai, Beijing 100192 (CN); TANG, Aolin, Beijing 100192 (CN)
(74) Representative: karo IP
(86) International application number: PCT/CN2022/074243
(87) International publication number: WO 2022/170988

(57) **Abstract**

The present disclosure relates to the field of instruments, and discloses a guidable and growable instrument and a surgical robot system. The guidable and growable instrument comprises: a growable tube comprising an inner layer, an outer layer, and a fluid cavity between the inner layer and the outer layer, the fluid cavity being configured to accommodate a fluid. The growable tube comprises a turnable region at a distal end, the inner layer and the outer layer are connected and turnable in the turnable region, a guide is arranged in a channel surrounded by the inner layer of the growable tube, and the distal end of the guide is bendable to drive the growable tube to turn. The guidable and growable instrument can better adapt to a gradually narrowed bent and complex orifice so as to reduce or avoid touch and friction with the orifice.

## Description

### Cross-reference to Relevant Applications

The present application claims the priority of a Chinese Patent Application with Application No. 2021101783776, filed on February 9, 2021 and entitled "Guidable and Growable Instrument and Surgical Robot System", the full disclosure of which is incorporated herein by reference in its entirety.

### Technical Field

The present disclosure relates to the field of instruments, and in particular to a guidable and growable instrument and a surgical robot system.

### Background

Traditional disease diagnosis and surgical treatment are mainly divided into open diagnosis and surgery and intracavitary interventional diagnosis and treatment. Intracavitary interventional diagnosis or treatment is to make an incision on blood vessels and skin to form a channel without performing surgical operation to expose the lesion, or to reach a target position through an original orifice of a human body under guidance of imaging device, so as to diagnose or treat the lesion locally, with the characteristics of less trauma.

Traditional intracavitary interventional surgery mainly relies on surgeons to perform manual operations. In order to reduce the burden on the surgeons and improve the efficiency and safety of intracavitary intervention, a method of using intracavitary interventional instruments to assist interventional diagnosis or surgery has gradually become a research hotspot in the industry. The intracavitary interventional instruments have the advantages of precise motion, high precision in repeated of positioning and being able to be manipulated remotely, as well as eliminating the danger resulting from physiological trembling and fatigue misoperation of surgeon during manual operation.

However, the currently employed methods of instrument-assisted interventional diagnosis or surgery have the following problems: 1. the interventional instruments have relatively large volume, limiting the further promotion of instrument-assisted intracavitary interventional diagnosis or surgery; 2. the interventional instruments have relatively poor flexibility, and they cannot adapt to the complex and bent orifice of human body, and will cause damage to the orifice.

### Summary of the Invention

Based on the above problems, the present disclosure provides a guidable and growable instrument and a surgical robot system, which have good flexibility, can achieve controllable growth and extension, and can well adapt to gradually narrowed bent and complex orifice.

In some embodiments, the present disclosure provides a guidable and growable instrument comprising: a growable tube comprising an inner layer, an outer layer, and a fluid cavity between the inner layer and the outer layer, the fluid cavity being configured to accommodate a fluid, wherein the growable tube comprises a turnable region at a distal end, the inner layer and the outer layer are connected and turnable in the turnable region; a guide arranged in a channel surrounded by the inner layer of the growable tube, wherein the distal end of the guide is bendable to drive the growable tube to turn.

In some embodiments, the present disclosure provides a surgical robot system comprising a system controller and the guidable and growable instrument as described above, wherein the system controller is configured to control the motion of the guidable and growable instrument.

### Brief Description of the Drawings

In order to explain the technical solutions in the embodiments of the present disclosure more clearly, the accompanying drawings required to be used in the description of the embodiments of the present disclosure will be briefly introduced below. Obviously, the accompanying drawings in the following description only show some of the embodiments of the present disclosure, and for those of ordinary skill in the art, other embodiments would also have been obtained from the contents of the embodiments of the present disclosure and these accompanying drawings without involving any inventive effort.
FIG. 1 shows a structural schematic diagram of a distal portion of a guidable and growable instrument according to some embodiments of the present disclosure;
FIG. 2 shows a structural schematic diagram of the distal portion of the guidable and growable instrument located within an orifice within a body according to some embodiments of the present disclosure;
FIG. 3(a) shows a cross-sectional view of a growable tube according to some embodiments of the present disclosure;
FIG. 3(b) shows another cross-sectional view of a growable tube according to some embodiments of the present disclosure;
FIG. 4(a) shows a schematic diagram of a distal structure of a gradually-changing growable tube according to some embodiments of the present disclosure;
FIG. 4(b) shows a schematic diagram of a distal structure of another gradually-changing growable tube according to some embodiments of the present disclosure;
FIG. 5(a) shows a schematic diagram of a distal structure of a stepwise-changing growable tube according to some embodiments of the present disclosure;
FIG. 5(b) shows a schematic diagram of a distal structure of another stepwise-changing growable tube according to some embodiments of the present disclosure;
FIG. 6(a) shows a structural schematic diagram of a part of a tube driving mechanism according to some embodiments of the present disclosure;
FIG. 6(b) shows a structural schematic diagram of a part of another tube driving mechanism according to some embodiments of the present disclosure;
FIG. 7 shows a structural schematic diagram of the guidable and growable instrument according to some embodiments of the present disclosure;
FIG. 8 shows a structural schematic diagram of another guidable and growable instrument according to some embodiments of the present disclosure;
FIG. 9 shows a structural schematic diagram of a guide according to some embodiments of the present disclosure;
FIG. 10(a) shows a structural schematic diagram of a guide driving mechanism according to some embodiments of the present disclosure;
FIG. 10(b) shows a structural schematic diagram of another guide driving mechanism according to some embodiments of the present disclosure;
FIG. 11 shows a structural schematic diagram of another guide according to some embodiments of the present disclosure;
FIG. 12 shows a structural schematic diagram of another guide according to some embodiments of the present disclosure;
FIG. 13 shows a structural schematic diagram of a turning member of the guide according to some embodiments of the present disclosure;
FIG. 14 shows a structure diagram of a bending unit according to some embodiments of the present disclosure;
FIG. 15 shows a structural schematic diagram of another turning member of the guide according to some embodiments of the present disclosure;
FIG. 16 shows a structural schematic diagram of a slit unit according to some embodiments of the present disclosure;
FIG. 17 shows a longitudinal cross-sectional view of another turning member of the guide according to some embodiments of the present disclosure.

### Detailed Description

To make the solved technical problems, used technical solutions, and achieved technical effects of the present disclosure more clearly, the technical solutions of the embodiments of the present disclosure will be further described in detail below with reference to the accompanying drawings. Obviously, the described embodiments are only a subset of embodiments, but not all of embodiments, of the present disclosure. Based on the embodiments in the present disclosure, all other embodiments obtained by those skilled in the art without doing creative work will fall within the scope of the present disclosure.

In the description of the present disclosure, it should be noted that, orientational or positional relationships indicated by the terms "center", "upper", "lower", "left", "right", "vertical", "horizontal", "inner", "outer" and the like are the orientational or positional relationships shown based on the accompanying drawings, and are only for ease of describing the present disclosure and simplifying the description, rather than indicating or implying that the apparatus or element referred to must have a specific orientation or be constructed and operated in a specific orientation, and therefore cannot be construed as limiting the present disclosure. In addition, the terms "first" and "second" are used for descriptive purposes only, and cannot be understood as indicating or implying relative importance. In the description of the present disclosure, it should be noted that, unless otherwise specified and defined, the term "mount", "connected", and "connect", or "couple" should be comprehended in a broad sense. For example, the term may be a fixed connection or a detachable connection; or may be a mechanical connection or an electrical connection; may be a direct connection or an indirect connection via an intermediate medium, or may be internal communication between two elements. For those of ordinary skill in the art, specific meanings of the foregoing terms in the present disclosure may be understood based on specific situations.

In the present disclosure, an end close to an operator (e.g., a surgeon) is defined as a proximal end, a proximal portion, a rear end, or a rear portion, and an end close to a patient who requires surgery is defined as a distal end, a distal portion, a front end, or a front portion. Those skilled in the art may understand that the guidable and growable instrument according to the embodiments of present disclosure may be used in the medical field, and may also be used in other non-medical fields.

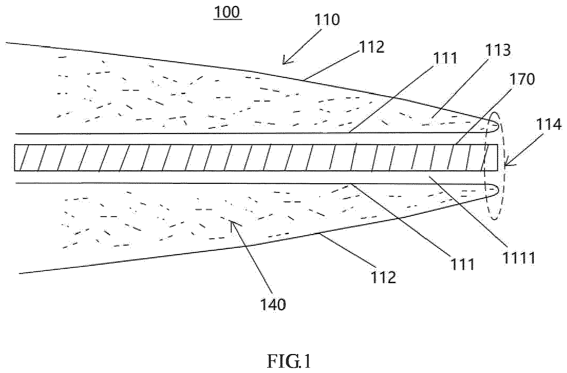

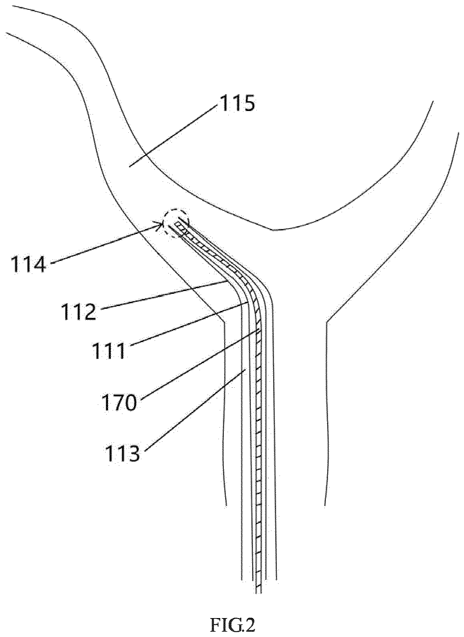

FIG. 1 shows a structural schematic diagram of a distal portion of a guidable and growable instrument 100 according to some embodiments of the present disclosure, and FIG. 2 shows a structural schematic diagram of the distal portion of the guidable and growable instrument 100 located within an orifice 115 (e.g., a blood vessel, a trachea, an esophagus, a vagina, an intestine, etc.) within a body (e.g., within a human body or animal body) according to some embodiments of the present disclosure. The guidable and growable instrument 100 may enter the orifice 115 through an opening (e.g., an incision or a natural opening). As shown in Figs. 1 and 2, the guidable and growable instrument 100 may comprise a growable tube 110. The growable tube 110 may comprise flexible material. The growable tube 110 comprises an inner layer 111, an outer layer 112, and a fluid cavity 113 between the inner layer 111 and the outer layer 112. The fluid cavity 113 is used to accommodate a fluid 140. The growable tube 110 also includes a turnable region 114 at a distal end, the inner layer 111 and the outer layer 112 are connected and bendable in the turnable region 114. In some embodiments, a radial dimension of a proximal end of the outer layer 112 is greater than the radial dimension of a distal end of the outer layer 112, as shown in Fig. 1. Those skilled in the art should understand that, in some embodiments, the radial dimension of the proximal end of the outer layer 112 may be equal to or smaller than the radial dimension of the distal end of the outer layer 112. The inner layer 111 can be turned outward at the turnable region 114 to form the outer layer 112, or the outer layer 112 can be turned inward at the turnable region 114 to form the inner layer 111. Through the turning between the inner layer 111 and the outer layer 112, the growable tube 110 can grow toward a distal end (e.g., extended or stretched) or withdraw, so that the guidable and growable instrument 100 grows to reach a target position in the orifice 115 or withdraw from the orifice 15. For example, the inner layer 111 moves distally by a length L, and the inner layer 111 with the length L turns outward in the turnable region 114 to form the outer layer 112, and the fluid 140 fills the fluid cavity 113 grown by the outward turning of the inner layer 111, thereby the growable tube 110 can grow forward. The inner layer 111 moves proximally by a length L', and the outer layer 112 with the length L' turns inward in the turnable region 114 to form the inner layer 111, thereby the growable tube 110 can withdraw.

In some embodiments, the guidable and growable instrument 100 may also include a guide 170 that can turn in at least one degree of freedom at the distal end. The inner layer 111 of the growable tube 110 surrounds to form a channel 1111, and the guide 170 is provided within the channel 1111, and when bending and turning, the distal end of the guide 170 can drive the growable tube 110 to turn. Through a bending and turning guidance of the guide 170, the turning of the growable tube 110 may be achieved to adapt to a bent and complex orifice 115. Thus, the growable tube 110 can grow distally, through the orifice 115, and grow to a target position. In some embodiments, the radial dimension of the proximal end of outer layer 112 may be larger than the radial dimension of the distal end of outer layer 112. In this manner, the guidable and growable instrument 100 is able to accommodate the gradually narrowed orifice 115 so as to reduce or avoid touch and friction with the orifice 115. In some embodiments, the guide 170 may include an endoscope, surgical end effector, drug delivery device located at the distal end, etc..

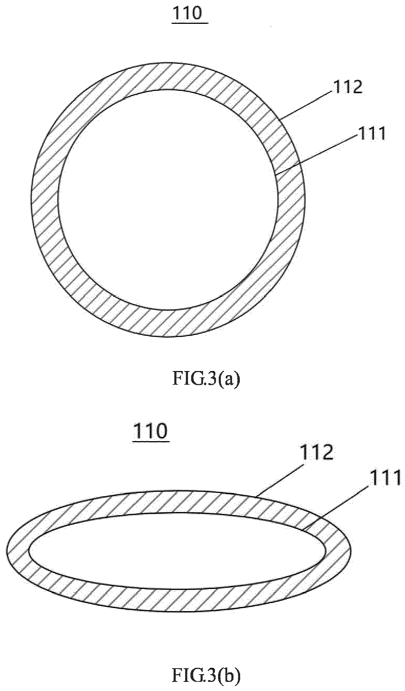

Figs. 3(a) and 3(b) respectively show cross-sectional views of the growable tube 110 according to some embodiments of the present disclosure. In some embodiments, as shown in FIG. 3(a), the growable tube 110 may have a circular cross-section. In some embodiments, as shown in FIG. 3(b), the growable tube 110 may have an oval cross-section. It should be understood that the cross-section of the growable tube 110 includes, but is not limited to, the structures of the above embodiments and may include other shapes, such as rectangular, polygonal, etc. In some embodiments, the growable tube 110 comprises flexible material including, but not limited to, plastic, rubber, etc., for example, low density polyethylene, silicon-containing polymers, or fluoropolymer, etc.. The flexible growable tube 110 can avoid injury to the orifice 115.

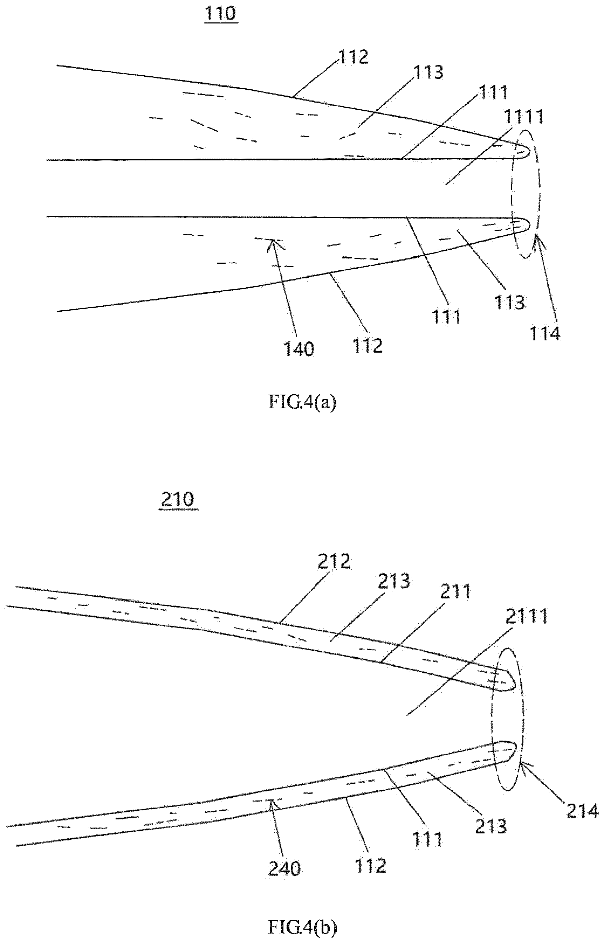

Fig. 4(a) and Fig. 4(b) respectively show the structural schematic diagrams of the distal portions of the gradually-changing growable tubes 110 and 210 according to some embodiments of the present disclosure. As shown in FIG. 4(a), in some embodiments, the radial dimension of the outer layer 112 may gradually decrease along an extension direction from the proximal end to the distal end. The contour of the outer layer 112 may be straight, curved, or a combination thereof. It can be understood that the shape and status of the growable tubes 110 and 210 shown in Fig. 4(a) and Fig. 4(b) may be the shape and status during the growth process or the shape and status when the growth stops. The inner layer 111 of the growable tube 110 may remain substantially constant along the extension direction from the proximal end to the distal end. In a state where the turning stops (e.g., a fully grown state, or when approaching a lesion), a thickness of the fluid cavity 113 gradually decreases along the extension direction from the proximal end to the distal end. The inner layer 111 surrounds to form the channel 1111, the radial dimension of the channel 1111 remain substantially constant along the extension direction from the proximal end to the distal end, and the channel 1111 may be used to accommodate the guide 170. Either the inner layer 111 or the outer layer 112 may be driven to move distally or proximally. For example, the inner layer 111 moves distally by a length L, and the inner layer 111 with the length L turns outward in the turnable region 114 to form the outer layer 112, and the fluid 140 fills the fluid cavity 113 grown by the outward turning of the inner layer 111, thereby, the growable tube 110 can grow forward. The inner layer 111 moves proximally by a length L', and the outer layer 112 with the length L' turns inward in the turnable region 114 to form the inner layer 111, thereby the growable tube 110 can withdraw.

As shown in FIG. 4(b), the radial dimension of the outer layer 212 may gradually decrease along the extension direction from the proximal end to the distal end. The contour of the outer layer 212 may be straight, curved, or a combination thereof. The inner layer 211 of the growable tube 210 may gradually decrease along the extension direction from the proximal end to the distal end. In a state where the turning stops (such as a fully grown state, or when approaching the lesion), the thickness of the fluid cavity 213 remains substantially constant or gradually decreases along the extension direction from the proximal end to the distal end. The inner layer 211 surrounds to form a channel 2111, and the radial dimension of the channel 2111 gradually decreases along the extension direction from the proximal end to the distal end. The channel 2111 may be used to accommodate the guide 170. Either the inner layer 111 or the outer layer 112 may be driven to move distally or proximally, such that the inner layer 211 can be turned outward in the turnable region 214 to form the outer layer 212, or the outer layer 212 can be turned inward in the turnable region 214 to form the inner layer 211.

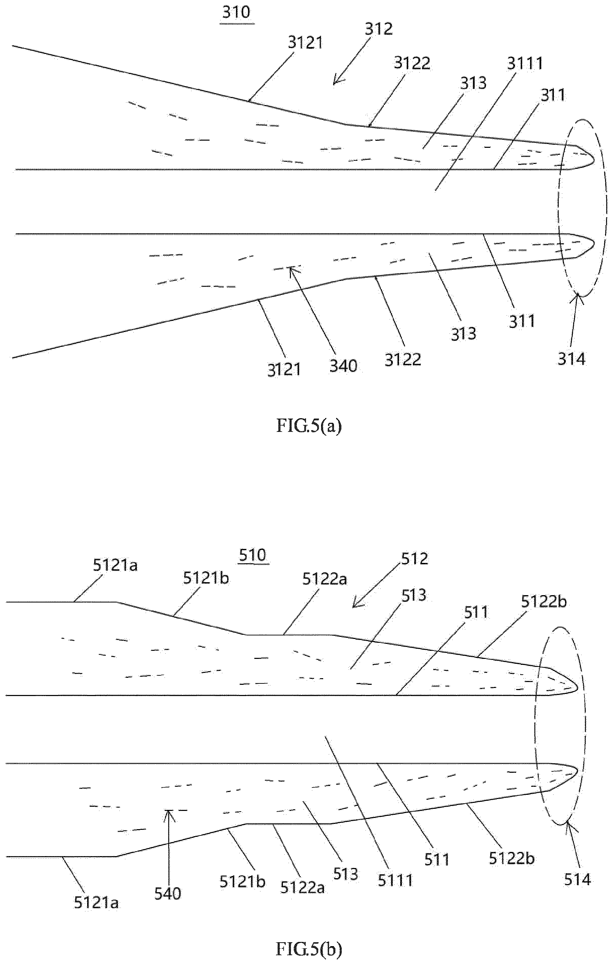

Fig. 5(a) and Fig. 5(b) respectively show the structural schematic diagrams of the distal portions of the stepwise-changing growable tubes 310 and 510 according to some embodiments of the present disclosure. In some embodiments, as shown in Fig. 5 (a) and Fig. 5 (b), the radial dimensions of the outer layers 312 and 512 may be decreased in a stepwise manner along the extension direction from the proximal end to the distal end. In the present disclosure, the stepwise manner refers to that a significant change of a contour slope of a layer appears occurs at a stepped region. It can be understood that the shapes and status of the growable tubes 310 and 510 shown in Fig. 5(a) and Fig. 5(b) can be the shapes and status during the growth process or the shapes and status when the growth stops. In some embodiments, as shown in Fig. 5 (a), the outer layer 312 may include a proximal segment 3121 and a distal segment 3122. A radial dimension of the proximal segment 3121 gradually decreases along the extension direction from the proximal end to the distal end, and the radial dimension of the distal segment 3122 gradually decreases along the extension direction from the proximal end to the distal end. The contour slope of the proximal segment 3121 at a connection is different from that of the distal segment 3122 at the connection, to form a stepwise-changing contour. The inner layer 311 of the growable tube 310 may remain substantially constant along the extension direction from the proximal end to the distal end. In a state where the turning stops (such as a fully grown state, or when approaching the lesion), the thickness of the fluid cavity 313 decreases in the stepwise manner along the extension direction from the proximal end to the distal end. The inner layer 311 surrounds to form the channel 3111, the radial dimension of the channel 3111 remain substantially constant along the extension direction from the proximal end to the distal end, and the channel 3111 is used to accommodate the guide 170. Either the inner layer 311 or the outer layer 312 may be driven to move distally or proximally. For example, the inner layer 311 moves distally by a length L, and the inner layer 311 with the length L turns outward in the turnable region 314 to form the outer layer 312, and the fluid 340 fills the fluid cavity 313 grown by the outward turning of the inner layer 311, thereby, the growable tube 310 can grow forward. The inner layer 311 moves proximally by a length L', and the outer layer 312 with the length L' turns inward in the turnable region 314 to form the inner layer 311, thereby the growable tube 310 can withdraw.

In some embodiments, as shown in FIG. 5 (b), the outer layer 512 may include a stepwise-changing contour composed of multiple segments with different radial dimensions. As shown in FIG. 5(b), the outer layer 512 may include in sequence a proximal segment 5121a, a proximal segment 5121b, a distal segment 5122a and a distal segment 5122b with different radial dimensions, the radial dimensions of the proximal segment 5121a and the distal segment 5122a remain substantially unchanged, and the radial dimensions of the proximal segment 5121b and the distal segment 5122b gradually decrease along the extension direction from the proximal end to the distal, respectively. The proximal segment 5121a and the proximal segment 5121b may be connected with each other at a connection region in a gradually-changing manner or in a suddenly-changing manner, the proximal segment 5121b and the distal segment 5122a may be connected with each other at a connection region in the gradually-changing manner or in the suddenly-changing manner, and the distal segment 5122a and the distal segment 5122b may be connected with each other at a connection region in the gradually-changing manner or in the suddenly-changing manner, to form a multi-segment stepwise-changing contour. The radial dimension of inner layer 511 of the growable tube 510 may remain substantially constant or gradually decrease along the extension direction from the proximal end to the distal end. In a state where the turning stops (such as a fully grown state, or when approaching the lesion), the thickness of the fluid cavity 513 decreases along the extension direction of the proximal segment 5121a, the proximal segment 5121b, the distal segment 5122a and the distal segment 5122b. The inner layer 511 surrounds to form the channel 5111, the radial dimension of the channel 5111 remain substantially constant from the proximal end to the distal end along the extending direction, and the channel 5111 is used to accommodate the guide 170. Either the inner layer 511 or the outer layer 512 may be driven to move distally or proximally, such that the inner layer 511 can be turned outward in the turnable region 514 to form the outer layer 512, or the outer layer 512 can be turned inward in the turnable region 514 to form the inner layer 511.

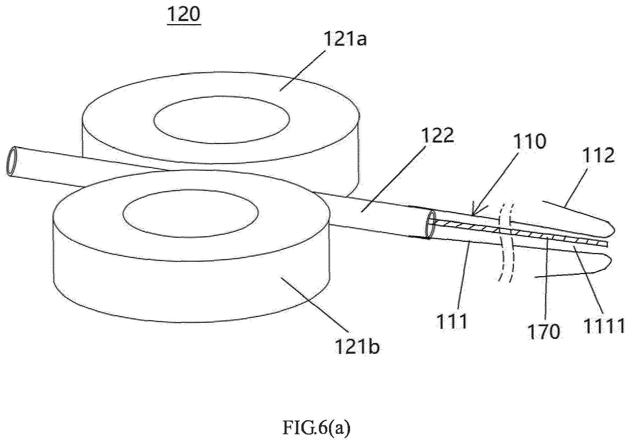

The guidable and growable instrument 100 may include one of the growable tubes 110, 210, 310, 510. In some embodiments, the guidable and growable instrument 100 may also include a tube driving-mechanism 120. FIG. 6(a) shows a structural schematic diagram of a part of the tube driving mechanism 120 according to some embodiments of the present disclosure. As shown in FIG. 6(a), the tube driving mechanism 120 is connected to the growable tube 110 (or 210, 310, 510), and the tube driving mechanism 120 can move linearly to drive the outer layer 112 or the inner layer 111 of the growable tube 110 to move. In some embodiments, the tube driving mechanism 120 may be connected to the outer layer 112 of the growable tube 110, thereby driving the outer layer 112 of the growable tube 110 to move. In some embodiments, as shown in FIG. 6(a), the tube driving mechanism 120 may be connected to the inner layer 111 of the growable tube 110 to drive the inner layer 111 of the growable tube 110 to move.

In some embodiments, as shown in FIG. 6 (a), the tube driving mechanism 120 may include two rollers 121a and 121b arranged in parallel, a moving rod 122 disposed between the two rollers 121a-b, and a driving unit (not shown in the figure) connected to the two rollers 121a-b respectively. The inner layer 111 of the growable tube 110 (or 210, 310, 510) is sealingly connected to the outer periphery of a distal end of the moving rod 122. The driving unit respectively drives the two rollers 121a-b to synchronously rotate in opposite directions at the same speed to drive the moving rod 122 to move linearly, so that the inner layer 111 of the growable tube 110 is driven to move through the moving rod 122. The moving rod 122 drives the inner layer 111 to move distally. In the turnable region 114, the inner layer 111 turns outward to form the outer layer 112, so that the fluid 140 fills the fluid cavity 113 that grows with the inner layer 111 turning outward. In some embodiments, the distance for which the growable tube 110 extends originating from the inner layer 111 turning outward is about the same as the distance for which the moving rod 122 moves. In some embodiments, the distance for which the growable tube 110 extends originating from the inner layer 111 turning outward is less than the distance for which the moving rod 122 moves.

In some embodiments, as shown in FIG. 6 (a), the guide 170 is disposed within the channel 1111, the proximal end of the guide 170 passes through an inner lumen of the moving rod 122, and is connected with a guide driving mechanism (not shown in the figure), and the guide 170 moves distally under the drive of the guide driving mechanism to synchronize with the growth of the growable tube 110. The distal end of the guide 170 may turn under the drive of the guide driving mechanism, thereby driving the growable tube 110 to turn. Through the guide 170, the turning of the growable tube 110 may be achieved to adapt to a bent and complex orifice 115.

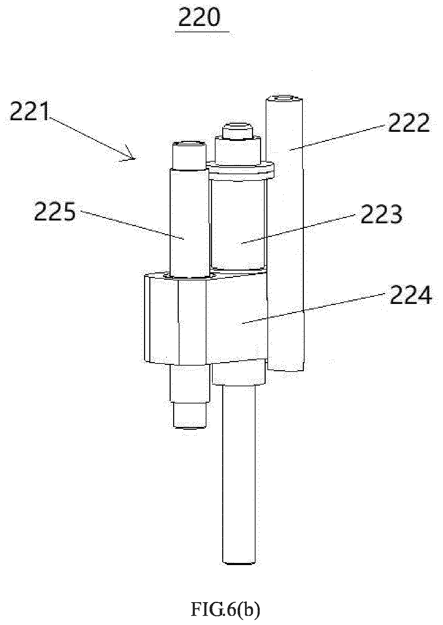

The guidable and growable instrument 200 may include a tube driving mechanism 220 and one of the growable tubes 110, 210, 310, 510. FIG. 6(b) shows a structural schematic diagram of a part of the tube driving mechanism 220 according to some embodiments of the present disclosure. In some embodiments, as shown in FIG. 6(b), the tube driving mechanism 220 may include a screw slider module 221 and a moving rod 222 driven by the screw slider module 221. The screw slider module 221 may include a lead screw 223 and a slider 224 connected by threads, the moving rod 222 fixedly connected with the slider 224 and a driving unit (not shown in the figure) connected with the lead screw 223. In some embodiments, the screw slider module 221 may further include a guide rod 225 disposed in the slider 224 by sliding through the slider 224. The outer layer 112 or the inner layer 111 of the growable tube 110 (or 210-610) is sealingly connected with the moving rod 222. The driving unit drives the lead screw 223 to rotate, the slider 224 may move linearly along the guide rod 225, and drives the moving rod 222 fixedly connected to the slider 224 to move linearly, thereby driving the outer layer 112 or the inner layer 111 of the growable tube 110 to move.

It should be understood that the tube driving mechanism of the present disclosure includes but is not limited to the structures of the above embodiments, any driving mechanism, as long as capable of realizing linear motion, does not depart from the scope of the present disclosure.

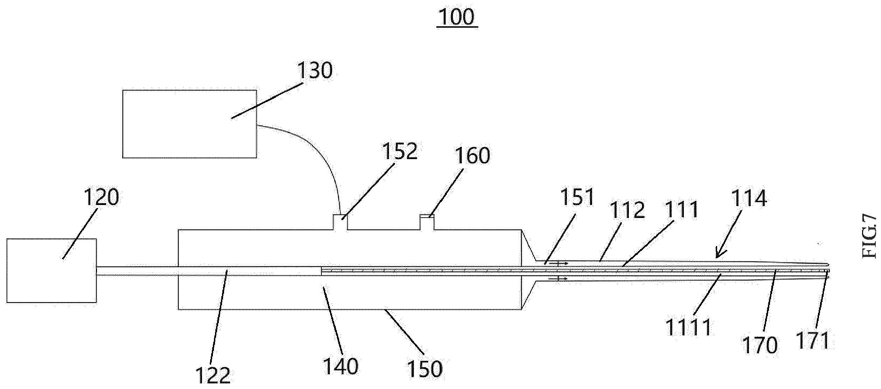

FIG. 7 shows a structural schematic diagram of the guidable and growable instrument 100 (or 200) according to some embodiments of the present disclosure. As shown in FIG. 7, in some embodiments, the guidable and growable instrument 100 (or 200) further comprises a fluid controller 130. The fluid controller 130 is used to pressurize the fluid 140 to drive the fluid 140 to gradually fill the fluid cavity 113 between the outer layer 112 and the inner layer 111. In some embodiments, the fluid 140 may be a liquid fluid, such as saline, or a gaseous fluid, such as air, carbon dioxide gas or other inert gas. In some embodiments, the fluid controller 130 may include a gas pump or a liquid pump and the like.

In some embodiments, as shown in FIG. 7, the guidable and growable instrument 100 (or 200) further comprises a fluid tank 150. The fluid tank 150 may include a fluid outlet channel 151 and a fluid control channel 152, and the fluid controller 130 communicates with the fluid tank 150 through the fluid control channel 152. The control channel 152 may include fluid catheters, switches and the like. In some embodiments, the outer layer 112 of the growable tube 110 may be sealingly connected to the outer periphery of the fluid outlet channel 151, the inner layer 111 of the growable tube 110 may extend through the fluid outlet channel 151 to a proximal end of the fluid tank 150 and may be sealingly connected with the moving rod 122 of the tube driving mechanism 120. The tube driving mechanism 120 may drive the inner layer 111 of the growable tube 110 to move distally or proximally. The fluid controller 130 may control the fluid pressure in the fluid tank 150 and the fluid cavity 113, for example, maintain the pressure in the fluid tank 150 and the fluid cavity 113 at a predetermined value or within a predetermined interval. In some embodiments, when the outer layer 112 or inner layer 111 is driven by the tube driving mechanism 120 to turn in the turnable region 114, the fluid controller 130 may control the fluid 140 to fill the growing fluid cavity 113 or retract from the withdrawing fluid cavity 113. For example, the tube driving mechanism 120 drives the inner layer 111 of the growable tube 110 to move distally by a length L, the inner layer 111 turns outwards in the turnable region 114 by the length L to form the outer layer 112, such that the fluid cavity 113 grows distally. The fluid controller 130 pressurizes (e.g., injects fluids into) the fluid tank 150, such that the fluid 140 fills into the fluid cavity 113 of the growable tube 110, thereby filling the fluid cavity 113 growing in the turnable region 114. As another example, the tube driving mechanism 120 drives the inner layer 111 of the growable tube 110 to move proximally by a length L', the outer layer 112 turns inwards in the turnable region 114 by the length L' to form the inner layer 111, such that the fluid cavity 113 withdraws proximally. The fluid controller 130 depressurizes (e.g., extracts fluids from) the fluid tank 150, such that the fluid 140 withdraws from the fluid cavity 113 of the growable tube 110 and into the fluid tank 150, thereby the growable tube 110 withdraws proximally. In some embodiments, the distance for the turning to grow or withdrawing is substantially equal to the distance traveled by the tube driving mechanism 120. In some embodiments, the distance for the turning to grow or withdrawing is less than the distance traveled by the tube driving mechanism 120.

In some embodiments, the tube driving mechanism 120 may be disposed outside of the fluid tank 150, the inner layer 111 of the growable tube 110 may extend through the fluid tank 150 and connect to the tube driving mechanism 120. In some embodiments, as shown in FIG. 7, at least part of the moving rod 122 of the tube driving mechanism 120 may be disposed inside of the fluid tank 150 and connected with the inner layer 111 of the growable tube 110.

In some embodiments, the guide 170 is disposed in the channel 1111, the proximal end of the guide 170 is connected to the guide driving mechanism (not shown in the figure) through the inner lumen of the moving rod 122 of the tube driving mechanism 120, and the distal end of the guide 170 bends and turns under the drive of the guide driving mechanism, thereby driving the growable tube 110 to turn.

In some embodiments, the guidable and growable instrument 100 further comprises a system controller (not shown in the figure), by which the distance travelled by the tube driving mechanism 120 and the pressure applied by the fluid controller 130 within the fluid cavity 113 are controlled, so that the guidable and growable instrument 100 may grow controllably. In some embodiments, the system controller may control the fluid controller 130, for example, sending pressurization and depressurization instructions to the fluid controller 130. In some embodiments, the system controller may also control the bending and turning of the guide 270 in order to control the growth direction of the guidable and growable instrument 200.

As shown in FIG. 7, in some embodiments, the guidable and growable instrument 100 further comprises a pressure sensor 160. The pressure sensor 160 may be disposed on the fluid tank 150 for detecting the pressure in the fluid tank 150. The pressure sensor 160 may be connected to the fluid controller 130 to send a fluid pressure signal within the fluid tank 150 to the fluid controller 130. The fluid controller 130 may control the fluid pressure in the fluid cavity 150 and the fluid cavity 113 according to the fluid pressure signal.

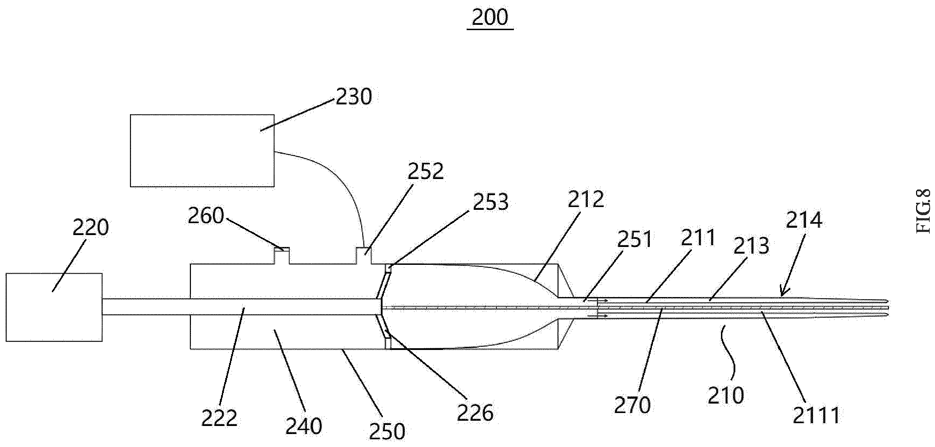

FIG. 8 shows a structural schematic diagram of the guidable and growable instrument 200 (or 100) according to some embodiments of the present disclosure. In some embodiments, as shown in FIG. 8, the guidable and growable instrument 200 (or 100) may also include a fluid tank 250. The fluid tank 250 includes a fluid outlet channel 251 and a fluid control channel 252, and the fluid controller 230 communicates with the fluid tank 250 through the fluid control channel 252. At least one sealing ring 253 may be provided within the fluid tank 250, and an outer periphery of the sealing ring 253 is sealingly fitted with the inner wall of the fluid tank 250. The fluid outlet channel 251 is annular. The inner layer 211 of the growable tube 210 is sealingly connected to the inside or outside of the inner ring wall of the fluid outlet channel 251. The outer layer 212 of the growable tube 210 extends through the fluid outlet channel 251 to the proximal end of the fluid tank 250 and is sealingly connected to the sealing ring 253. The sealing ring 253 and the moving rod 222 of the tube driving mechanism 220 are connected with each other by at least one connecting rod 226. At least part of the moving rod 222 of the tube driving mechanism 220 is disposed inside the fluid tank 250 and connected with the sealing ring 253 to drive the sealing ring 253 to move linearly along the length direction of the fluid tank 250. The sealing ring 253 may prevent the fluid 240 within the fluid tank 250 from leaking from the gap between the outer layer 212 of the growable tube 210 and the inner layer of the fluid tank 250. For example, the tube driving mechanism 220 drives the outer layer 212 of the growable tube 210 to move distally by a length L, and the outer layer 212 with the length L turns inward in the turnable region 214 to form the inner layer 211, and the fluid 240 fills the fluid cavity 213 grown by the inward turning of the outer layer 212, thereby, the growable tube 210 can grow forward. The outer layer 212 moves proximally by a length L' and the inner layer 211 with length L' turns outward in the turnable region 214 to form the outer layer 212, so that the growable tube 210 can withdraw. In some embodiments, as shown in FIG. 8, the pressure sensor 260 is disposed on the fluid tank 250 for detecting the pressure within the fluid tank 250. The pressure sensor 260 may be connected to the fluid controller 230 to send a fluid pressure signal within the fluid tank 250 to the fluid controller 230. The fluid controller 230 may control the fluid pressure in the fluid tank 250 and the fluid cavity 213 according to the fluid pressure signal.

In some embodiments, the guide 270 is disposed in the channel 2111, the proximal end of the guide 270 is connected to the guide driving mechanism (not shown in the figure) through the inner lumen of the moving rod 222 of the tube driving mechanism 220, and the distal end of the guide 270 bends and turns under the drive of the guide driving mechanism, thereby driving the growable tube 210 to turn. In some embodiments, the guidable and growable instrument 200 further comprises a system controller (not shown in the figure), by which the distance travelled by the tube driving mechanism 220 and the pressure applied by the fluid controller 230 within the fluid cavity 213 are controlled, so that the guidable and growable instrument 200 can work precisely. In some embodiments, the system controller may control the fluid controller 230, for example, sending pressurization and depressurization instructions to the fluid controller 230. In some embodiments, the system controller may also control the bending and turning of the guide 270 in order to control the growth direction of the guidable and growable instrument 200.

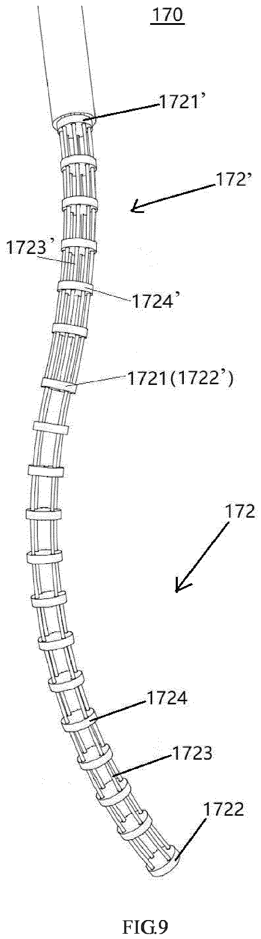

FIG. 9 shows a structural schematic diagram of a guide 170 according to some embodiments of the present disclosure. In some embodiments, as shown in FIG. 9, the guide 170 may include at least one distal continuum structure 172. The distal continuum structure 172 includes a distal base disk 1721, a distal stopping disk 1722, and a plurality of first structural backbones 1723. The distal base disk 1721 and the distal stopping disk 1722 are arranged at an interval, distal ends of the plurality of the first structural backbones 1723 are connected with the distal stopping disk 1722, and proximal ends of the plurality of the first structural backbones 1723 pass through the distal base disk 1721. In some embodiments, the distal ends of the plurality of first structural backbones 1723 are disposed on the distal stopping disk 1722 in a fastening fashion and at circumferential intervals. For example, the plurality of first structural backbones 1723 may be disposed at uniform spacings or disposed regularly and symmetrically. In some embodiments, the plurality of first structural backbones 1723 may be Nickel-titanium alloy wire, steel wire and the like. In some embodiments, the number of the first structural backbones 1723 may be 4, and by synergistically pushing or pulling two structural backbones disposed in correspondence, the bending and turning of the distal continuum structure 172 towards a first degree of freedom direction can be achieved, and by synergistically pushing or pulling the other two structural backbones disposed in correspondence, the bending and turning of the distal continuum structure 172 towards a second degree of freedom direction can be achieved, so that the guide 170 has a degree of freedom in at least one direction. In some embodiments, the number of the first structural backbones 1723 may also be 6, 8, 12 and the like. The number of the first structural backbones 1723 may include, but is not limited to, the number in the above embodiments.

As shown in FIG. 9, in some embodiments, the guide 170 may include distal continuum structures 172, 172' connected in series. The distal base disk 1721 of the distal continuum structure 172 located at the distal end becomes the distal stopping disk 1722' of the distal continuum structure 172' located at proximal end. By disposing two or more distal continuum structures 172, a flexibility for bending and turning of the guide 170 may be increased.

In some embodiments, as shown in FIG. 9, the distal continuum structures 172may also include at least one distal spacer disks 1724 disposed between the distal base disk 1721 and the distal stopping disk 1722, the distal continuum structures 172' may also include at least one distal spacer disks 1724' disposed between the distal base disk 1721' and the distal stopping disk 1722', the proximal ends of the plurality of first structural backbones 1723 pass sequentially through at least one distal spacer disk 1724, distal base disk 1721, distal spacer disk 1724' and distal base disk 1721', and the proximal ends of the plurality of first structural backbones 1723' pass sequentially through at least one distal spacer disk 1724' and distal base disk 1721'. By disposing the distal spacer disks 1724 and 1724', the stability of the plurality of first structural backbones 1723 and 1723' during the process of pushing or pulling can be enhanced.

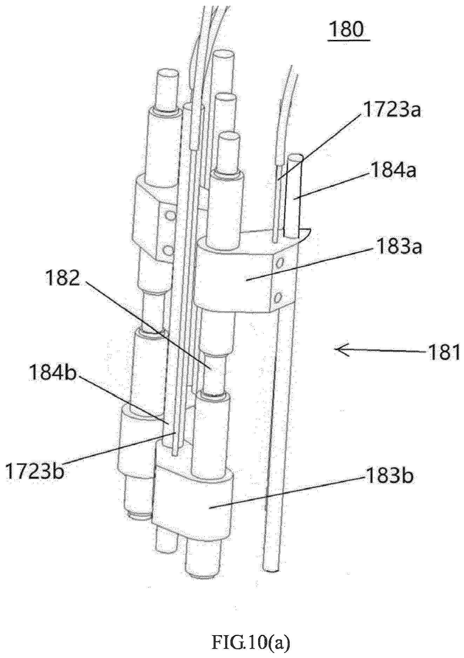

In some embodiments, the guidable and growable instrument 100 (or 200) may also include a guide driving mechanism, and the guide driving mechanism may be connected with the plurality of first structural backbones 1723, 1723', and drive the distal continuum structures 172, 172' to turn towards different directions in space by pushing or pulling the first structural backbones 1723, 1723'. FIG. 10(a) shows a structural schematic diagram of a guide driving mechanism 180 according to some embodiments of the present disclosure. As shown in FIG. 10(a), in some embodiments, the guide driving mechanism 180 may include at least one set of double-head lead screw module 181. The double-head lead screw module 181 may include a double-head lead screw 182, a pair of sliders 183a and 183b threaded with the double-head lead screw 182, and a driving unit (not shown in the figure) connected with the double-head lead screw 182. In some embodiments, the double-head lead screw module 181 may include guide rods 184a and 184b disposed in the sliders 183a and 183b by sliding through the sliders 183a and 183b, respectively. At least one pair of first structural backbones 1723a and 1723b are fixedly connected to the sliders 183a and 183b, respectively. The driving unit drives the double-head lead screw 182 to rotate, and drives the sliders 183a and 183b to linearly move synchronously and in opposite directions (e.g., along the guide rods 184a and 184b), so that the first structural backbones 1723a and 1723b can be pushed or pulled synergistically. With the at least one set of double-head lead screw module 181, the plurality of first structural backbones 1723 may be pushed or pulled synergistically to achieve the bending and turning of the distal continuum structures 172 or 172'.

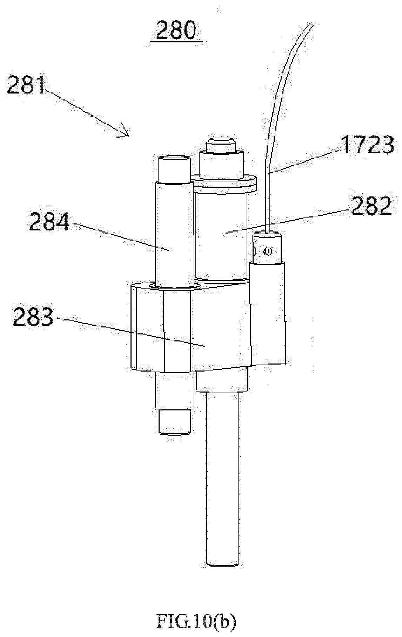

In some embodiments, the guidable and growable instrument 100 (or 200) may also include a guide driving mechanism. FIG. 10(b) shows a structural schematic diagram of a guide driving mechanism 280 according to some embodiments of the present disclosure. As shown in FIG. 10(b), the guide driving mechanism 280 may include at least one set of screw nut module 281. The screw nut module 281 may include a lead screw 282 and a nut 283 threaded with each other, a guide rod 284 disposed in the nut 283 by sliding through the nut 283 and a driving unit (not shown in the figure) connected with the lead screw 282. At least one first structural backbone 1723 or 1723' is fixedly connected with the nut 283. The driving unit drives the lead screw 282 to rotate, and drives the nut 283 to move linearly (e.g. along the guide rod 284), thereby pushing or pulling the first structural backbone 1723 or 1723'. With the at least one set of screw nut module 281, the plurality of first structural backbones 1723 or 1723' may be pushed or pulled synergistically to achieve the bending and turning of the distal continuum structures 172. It should be understood that the guide driving mechanism of the present disclosure includes but is not limited to the structures in the above embodiments, any driving mechanism, as long as capable of realizing the pushing or pulling of the structural backbone, does not depart from the scope of the present disclosure.

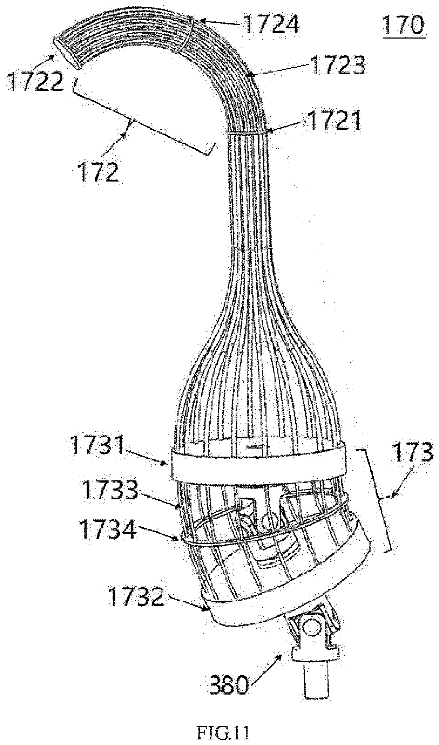

In some embodiments, as shown in FIG. 11, the guide 170 may also include at least one proximal continuum structure 173 including a proximal base disk 1731, a proximal stopping disk 1732 and a plurality of second structural backbones 1733. The proximal base disk 1731 and the proximal stopping disk 1732 are arranged at an interval, the proximal base disk 1731 is adjacent to the distal base disk 1721, the proximal ends of the plurality of second structural backbones 1733 are connected with the proximal stopping disk 1732, and the distal ends of the plurality of second structural backbones 1733 may pass through the proximal base disk 1731 and may be fixedly connected or integrated with the proximal ends of the plurality of first structural backbones 1723, respectively. In some embodiments, the plurality of second structural backbone 1733 may be Nickel-titanium alloy wire, steel wire and the like.

In some embodiments, as shown in FIG. 11, the proximal continuum structure 173 may also include at least one proximal spacer disk 1734 disposed between the proximal base disk 1731 and the proximal stopping disk 1732, and the proximal ends of the plurality of second structural backbones 1733 pass through the at least one proximal spacer disk 1734 and the proximal base disk 1731 in sequence. By disposing the proximal spacer disk 1734, the stability of the plurality of second structural backbones 1733 during the process of pushing or pulling can be enhanced.

In some embodiments, as shown in FIG. 11, the guide driving mechanism 380 may be connected with the proximal stopping disk 1732 for driving the proximal stopping disk 1732 to move and turn, thereby realizing the pushing or pulling of the second structural backbone 1733 disposed on the proximal stopping disk 1732, and through the second structural backbone 1733, the first structural backbone 1723 is pushed or pulled to drive the distal continuum structure 172 to turn in space along different directions. In some embodiments, the second structural backbone 1733 and the first structural backbone 1723 may be fixedly connected or the same structural backbone integrally formed. In some embodiments, the proximal ends of the plurality of second structural backbones 1733 are connected with the proximal stopping disk 1732 and pass through the proximal stopping disk 1732, and the guide driving mechanism 180 (or 280) may be connected with the plurality of second structural backbones 1733, and drive the distal continuum structure 172 to turn in space along different directions by synergistically pushing or pulling the plurality of second structural backbones 1733.

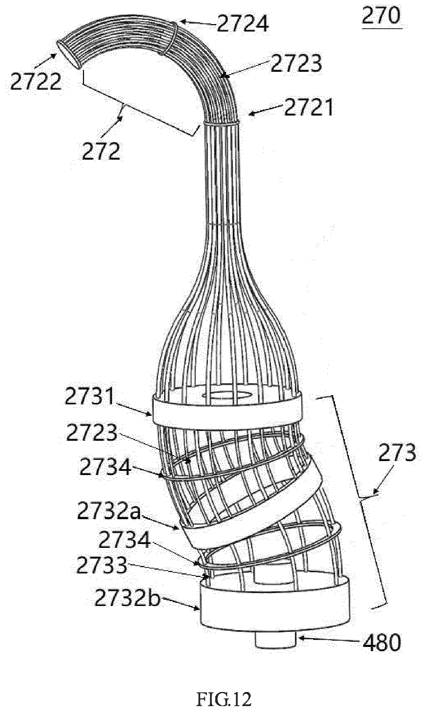

FIG. 12 shows a structural schematic diagram of a guide 270 according to some embodiments of the present disclosure. In some embodiments, as shown in FIG. 12, the guide 270 may also include at least one proximal continuum structure 273 including a proximal base disk 2731, a first proximal stopping disk 2732a, a second proximal stopping disk 2732b and a plurality of second structural backbones 2733. The proximal base disk 2731, the first proximal stopping disk 2732a and the second proximal stopping disk 2732b are arranged at intervals, the proximal base disk 2731 is adjacent to the distal base disk 2721, the proximal ends of the plurality of second structural backbones 2733 are connected with the second proximal stopping disk 2732b and the distal ends of the plurality of second structural backbones 2733 may pass through the first proximal stopping disk 2732a and are connected with the proximal base disk 2731, and the proximal ends of the plurality of first structural backbones 2723 pass through the proximal base disk 2731 and are connected with the first proximal stopping disk 2732a.

In some embodiments, the guide driving mechanism 480 may be connected with the second proximal stopping disk 2732b for driving the second proximal stopping disk 2732b to move and turn, causing the proximal base disk 2731 and the second proximal stopping disk 2732b to be misaligned, so that the plurality of second structural backbones 2733 are bent and turned, which drives the first proximal stopping disk 2732a to turn synergistically therewith, thereby pushing or pulling the plurality of first structural backbones 2723 with their ends being fixed on the first proximal stopping disk 2732a, and accordingly driving the distal continuum structure 272 to turn towards different directions in space. In some embodiments, the proximal ends of the plurality of second structural backbones 2733 are connected with the second proximal stopping disk 2732b and pass through the second proximal stopping disk 2732b, and connected with the guide driving mechanism 180 (or 280).The first proximal stopping disk 2732a turns synergistically by pushing or pulling the second structural backbone 2733, thereby pushing or pulling the plurality of first structural backbones 2723 to drive the distal continuum structure 272 to turn towards different directions in space.

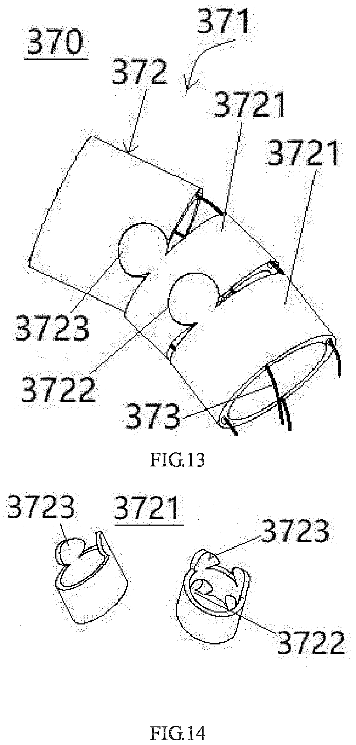

In some embodiments, the guide may include a turning member and driving wires connected with the bending member. FIG. 13 shows a structure diagram of a turning member 371 of the guide 370 according to some embodiments of the present disclosure. As shown in FIG. 13, a distal end of the driving wire 373 is connected with a distal end of the turning member 371, and the driving wire 373 may drive the turning member 371 to turn in at least one degree of freedom direction under the drive of the guide driving mechanism (e.g., the guide driving mechanism 180 or 280), thereby driving the growable tube 110 (or 210, 310, 510) to turn, so as to adapt to a bent and complex orifice 115.

In some embodiments, as shown in FIG. 13, the turning member 371 may include a snake bone structure 372. FIG. 14 shows a structural schematic diagram of a bending unit 3721 according to some embodiments of the present disclosure. As shown in FIGS. 13 and 14, the snake bone structure 372 may include a plurality of hollow bamboo-like bending units 3721 connected in head-to-tail manner, and through a connecting groove 3722 and a connecting protrusion 3723 connected with each other, two adjacent bending units 3721 may form a motion pair that can be bent and turned radially. The driving wire 373 may be disposed within and throughout various bending units 3721, or disposed within and through walls of various bending units 3721 (see FIG. 13). The distal end of the driving wire 373 is fixedly disposed at the distal end of the snake bone structure 372, and the guide driving mechanism pushes or pulls the driving wire 373 to drive the snake bone structure 372 to turn, thereby driving the growable tube 110 to turn. In some embodiments, there may be a plurality of the driving wires 373, and they are distributed circumferentially at intervals. The bending direction of the snake bone structure 372 may be adjusted by pushing, pulling, or synergistically pushing or pulling the plurality of driving wires 373, so as to achieve the bending and turning of the growable tube 110 towards a plurality of degree of freedom directions.

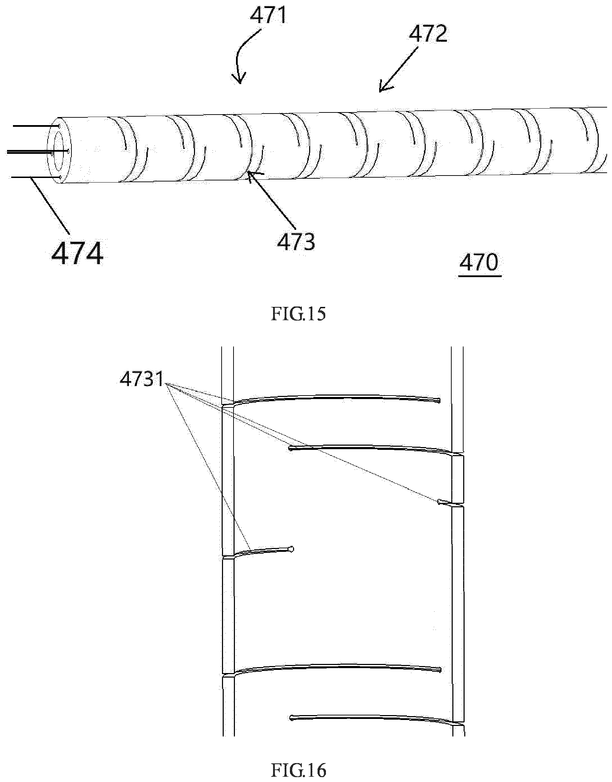

In some embodiments, the turning member may be a flexible sheath. FIG. 15 shows a structural schematic diagram of a turning member 471 of the guide 470 according to some embodiments of the present disclosure, FIG. 16 shows a structural schematic diagram of a slit unit 473 according to some embodiments of the present disclosure. As shown in FIGS. 15 and 16, the flexible sheath 472 may be provided with a plurality of slit units 473 along its extension direction at intervals. Each slit unit 473 may include at least one slit 4731 extending circumferentially along the flexible sheath 472. In some embodiments, the slit unit 473 may include a plurality of slits 4731, which are disposed along the axial direction of the flexible sheath 472 at intervals, and are disposed sequentially along the circumferential direction of the flexible sheath 472 in a misaligned way. The driving wire 474 may be disposed within and throughout the flexible sheath 472, or disposed within and throughout a wall of the flexible sheath 472 (see FIG. 15), and the distal end of the driving wire 474 is fixedly disposed at the distal end of the flexible sheath 472. The guide driving mechanism (e.g., the guide driving mechanism 180 or 280) pushes or pulls the driving wire 474 to drive the flexible sheath 472 to turn, thereby driving the growable tube 110 to turn. In some embodiments, there may be a plurality of driving wires 474, and they are distributed circumferentially at intervals. The bending direction of the flexible sheath 472 may be adjusted by pushing, pulling, or synergistically pushing or pulling the plurality of driving wires 474. In some embodiments, the flexible sheath 472 may rotate axially, so that the bending direction of the flexible sheath 472 can be adjusted to achieve the bending and turning of the growable tube 110 towards a plurality of degree of freedom directions.

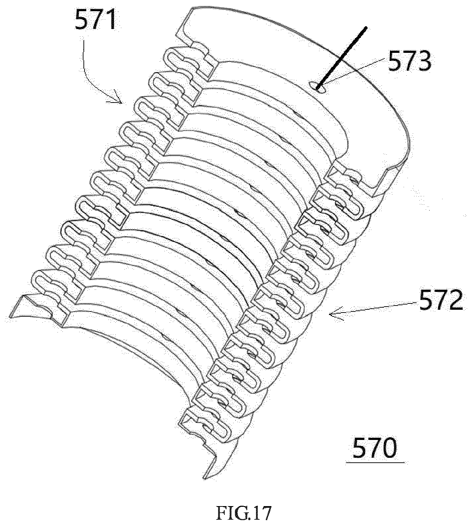

FIG. 17 shows a longitudinal cross-sectional view of a turning member 571 of the guide 570 according to some embodiments of the present disclosure. As shown in FIG. 17, in some embodiments, the turning member 571 may also include a bellows 572. The driving wire 573 may be disposed within and throughout the bellows 572, or disposed in and throughout a wall of the bellows 572 (see FIG. 17). The distal end of the driving wire 573 is fixedly disposed at a distal end of the bellows 572. The guide driving mechanism (e.g., the guide driving mechanism 180 or 280) pushes or pulls the driving wire 573 to drive the bellows 572 to turn, thereby driving the growable tube 110 (or 210, 310, 510) to turn. It should be understood that the turning member includes, but are not limited to, the above structures, and any structure that can be turned falls within the protection scope of the present disclosure.

The guidable and growable instrument 100 (or 200) may include any one of the guide 170-570. In some embodiments, as shown in FIG. 7, the guide 170 (or 270-570) may further include a medical instrument 171. The medical instrument 171 may be fixedly disposed at the distal end of the guide 170 and include, for example, an endoscope, a surgical end effector, an ultrasound probe, a probe, and the like. In some embodiments, the medical instrument 171 may be disposed in an internal channel of the guide 170, such as a drug delivery device that delivers drugs, and the like.

The present disclosure also provides a surgical robot system (not shown in the figure), which may include a system controller and the guidable and growable instrument 100 (or 200) as described above. The system controller is configured to control the motion of the guidable and growable instrument, for example, to control the growable tube 100 to grow or withdraw, or to control the guide 170 to advance, turn or withdraw. Because the guidable and growable instrument has good flexibility and can grow and extend in real time, it can better adapt to a bent and complex orifice, and can be widely used in the interventional diagnosis and treatment of various orifices.

This disclosure also discloses the following examples.
1. A guidable and growable instrument comprising:
   a growable tube comprising an inner layer, an outer layer, and a fluid cavity between the inner layer and the outer layer, the fluid cavity being configured to accommodate a fluid;
   wherein the growable tube comprises a turnable region at a distal end, the inner layer and the outer layer are connected and turnable in the turnable region;
   a guide arranged in a channel surrounded by the inner layer of the growable tube, wherein a distal end of the guide is bendable to drive the growable tube to turn.
2. The guidable and growable instrument according to item 1, wherein a radial dimension of a proximal end of the outer layer is greater than or equal to a radial dimension of a distal end of the outer layer.
3. The guidable and growable instrument according to item 1, wherein a radial dimension of the outer layer is substantially constant, gradually decreasing or decreasing in a stepwise manner along an extension direction from a proximal end to a distal end.
4. The guidable and growable instrument according to item 1, wherein a radial dimension of the inner layer remains constant or gradually decreases along an extension direction from a proximal end to a distal end.
5. The guidable and growable instrument according to item 1, wherein the guide comprises at least one distal continuum structure including a distal base disk, a distal stopping disk and a plurality of first structural backbones, wherein the distal base disk and the distal stopping disk are arranged at an interval, distal ends of the plurality of first structural backbones are connected with the distal stopping disk, and proximal ends of the plurality of first structural backbones pass through the distal base disk.
6. The guidable and growable instrument according to item 5, wherein the guide further comprises:
   at least one proximal continuum structure including a proximal base disk, a proximal stopping disk and a plurality of second structural backbones, wherein the proximal base disk and the proximal stopping disk are arranged at an interval, the proximal base disk is adjacent to the distal base disk, proximal ends of the plurality of second structural backbones are connected with the proximal stopping disk, and distal ends of the plurality of second structural backbones pass through the proximal base disk and are fixedly connected or integrated with the proximal ends of the plurality of first structural backbones, respectively.
7. The guidable and growable instrument according to item 5, wherein the guide further comprises:
   at least one proximal continuum structure including a proximal base disk, a first proximal stopping disk, a second proximal stopping disk and a plurality of second structural backbones, wherein the proximal base disk, the first proximal stopping disk and the second proximal stopping disk are arranged at intervals, the proximal base disk is adjacent to the distal base disk, proximal ends of the plurality of second structural backbones are fixedly connected with the second proximal stopping disk, and distal ends of the plurality of second structural backbones pass through the first proximal stopping disk and are connected with the proximal base disk;
   wherein the proximal ends of the plurality of first structural backbones pass through the proximal base disk and are connected with the first proximal stopping disk.
8. The guidable and growable instrument according to item 5, further comprising a guide driving mechanism, wherein the guide driving mechanism is connected with the plurality of first structural backbones, and drives the distal continuum structure to turn along different directions in space by pushing or pulling the first structural backbones.
9. The guidable and growable instrument according to items 6 or 7, further comprising a guide driving mechanism, wherein the guide driving mechanism is connected with the proximal stopping disk at the most proximal end and is configured to drive the proximal stopping disk to move and turn, thereby pushing or pulling the second structural backbones to drive the distal continuum structure to turn along different directions in space; or
   wherein the guide driving mechanism is connected with the plurality of second structural backbones, and drives the distal continuum structure to turn along different directions in space by pushing or pulling the second structural backbones.
10. The guidable and growable instrument according to item 1, wherein the guide comprises a turning member and driving wires disposed within the turning member, wherein distal ends of the driving wires are connected with a distal end of the turning member, and the driving wires are configured to drive the turning member to turn in at least one degree of freedom direction under the drive of the guide driving mechanism.
11. The guidable and growable instrument according to item 10, wherein the turning member is a snake bone structure including a plurality of hollow bamboo-like bending units connected in head-to-tail manner, and through a connecting groove and a connecting protrusion connected with each other, two adjacent bending units form a motion pair that is bendable radially.
12. The guidable and growable instrument according to item 10, wherein the turning member is a flexible sheath provided with a plurality of slit units along its extension direction at intervals, wherein each of the slit units includes at least one slit extending circumferentially along the flexible sheath.
13. The guidable and growable instrument according to item 10, wherein the turning member is a bellows, the driving wires can be disposed within and throughout the bellows, or disposed in and throughout a bellows wall of the bellows.
14. The guidable and growable instrument according to any one of items 1-8, further comprising: a tube driving mechanism connected to the growable tube, for driving the outer layer or the inner layer of the growable tube to move.
15. The guidable and growable instrument according to item 14, wherein the tube driving mechanism comprises: a driving unit, a moving rod, and a transmission unit connected with the driving unit and the moving rod, wherein the moving rod is sealingly connected with the inner layer or the outer layer of the growable tube, and the transmission unit is configured to convert rotary motion of the driving unit into linear motion, so as to drive the moving rod to drive the growable tube to grow or withdraw.
16. The guidable and growable instrument according to item 15, wherein the transmission unit comprises first and second rollers arranged in parallel;
   a proximal end of the moving rod is disposed between the first roller and the second roller and abuts against the first and second rollers, and a distal end of the moving rod is sealingly connected with the inner layer or the outer layer of the growable tube;
   the driving unit is connected with the first and second rollers, respectively, for driving the first and second rollers to synchronously rotate in opposite directions at the same speed, so as to drive the moving rod to move linearly.
17. The guidable and growable instrument according to item 15, wherein the transmission unit comprises a screw slider module including a lead screw and a slider connected by threads;
   the proximal end of the moving rod is fixedly connected with the slider, and the distal end of the moving rod is sealingly connected with the inner layer or the outer layer of the growable tube; and
   the driving unit is connected with the lead screw for driving the lead screw to rotate, so as to drive the moving rod to move linearly.
18. The guidable and growable instrument according to item 14, further comprising a fluid controller;
   wherein the fluid controller is configured to pressurize or depressurize the fluid, so as to drive the fluid to fill the fluid cavity of the turnable region or drive the fluid to withdraw from the fluid cavity.
19. The guidable and growable instrument according to any one of items 1-8, wherein the fluid is a liquid fluid or a gaseous fluid.
20. The guidable and growable instrument according to any one of items 1-8, wherein the growable tube is made of flexible material.
21. The guidable and growable instrument according to any one of items 1-8, wherein the cross-section of the growable tube is circular or oval.
22. The guidable and growable instrument according to any one of items 1-8, wherein the guide further comprises a medical instrument fixedly disposed at the distal end of the guide or disposed in an internal channel of the guide.
23. The guidable and growable instrument according to item 22, wherein the medical instrument includes an ultrasound probe, a probe, a drug capsule, or a surgical end effector.
24. A surgical robot system comprising a system controller and the guidable and growable instrument according to any one of items 1-23, wherein the system controller is configured to control a motion of the guidable and growable instrument.

Note that the above are only exemplary embodiments of the present disclosure and the applied technical principles. Those skilled in the art would appreciate that the present disclosure is not limited to specific embodiments herein, and those skilled in the art could make various apparent changes, readjustments and substitutions without departing from the scope of protection of the present disclosure. Thus, although the present disclosure is described in more detail by the above embodiments, the present disclosure is not limited to the above embodiments. Without departing from the concept of the present disclosure, more other equivalent embodiments may be included, and the scope of the present disclosure is determined by the scope of the appended claims.

## Claims

1. A guidable and growable instrument comprising:
a growable tube comprising an inner layer, an outer layer, and a fluid cavity between the inner layer and the outer layer, the fluid cavity being configured to accommodate a fluid;
wherein the growable tube comprises a turnable region at a distal end, the inner layer and the outer layer are connected and turnable in the turnable region;
a guide arranged in a channel surrounded by the inner layer of the growable tube, wherein a distal end of the guide is bendable to drive the growable tube to turn.

2. The guidable and growable instrument according to claim 1, wherein a radial dimension of a proximal end of the outer layer is larger than or equal to a radial dimension of a distal end of the outer layer.

3. The guidable and growable instrument according to claim 1, wherein a radial dimension of the outer layer is substantially constant, gradually decreasing or decreasing in a stepwise manner along an extension direction from a proximal end to a distal end.

4. The guidable and growable instrument according to claim 1, wherein a radial dimension of the inner layer remains constant or gradually decreases along an extension direction from a proximal end to a distal end.

5. The guidable and growable instrument according to claim 1, wherein the guide comprises at least one distal continuum structure including a distal base disk, a distal stopping disk and a plurality of first structural backbones, wherein the distal base disk and the distal stopping disk are arranged at an interval, distal ends of the plurality of first structural backbones are connected with the distal stopping disk, and proximal ends of the plurality of first structural backbones pass through the distal base disk.

6. The guidable and growable instrument according to claim 5, wherein the guide further comprises:
at least one proximal continuum structure including a proximal base disk, a proximal stopping disk and a plurality of second structural backbones, wherein the proximal base disk and the proximal stopping disk are arranged at an interval, the proximal base disk is adjacent to the distal base disk, proximal ends of the plurality of second structural backbones are connected with the proximal stopping disk, and distal ends of the plurality of second structural backbones pass through the proximal base disk and are fixedly connected or integrated with the proximal ends of the plurality of first structural backbones, respectively.

7. The guidable and growable instrument according to claim 5, wherein the guide further comprises:
at least one proximal continuum structure including a proximal base disk, a first proximal stopping disk, a second proximal stopping disk and a plurality of second structural backbones, wherein the proximal base disk, the first proximal stopping disk and the second proximal stopping disk are arranged at intervals, the proximal base disk is adjacent to the distal base disk, proximal ends of the plurality of second structural backbones are fixedly connected with the second proximal stopping disk, and distal ends of the plurality of second structural backbones pass through the first proximal stopping disk and are connected with the proximal base disk;
wherein the proximal ends of the plurality of first structural backbones pass through the proximal base disk and are connected with the first proximal stopping disk.

8. The guidable and growable instrument according to claim 5, further comprising a guide driving mechanism, wherein the guide driving mechanism is connected with the plurality of first structural backbones, and drives the distal continuum structure to turn along different directions in space by pushing or pulling the first structural backbones.

9. The guidable and growable instrument according to claim 6, further comprising a guide driving mechanism, wherein the guide driving mechanism is connected with the proximal stopping disk at the most proximal end and is configured to drive the proximal stopping disk to move and turn, thereby pushing or pulling the second structural backbones to drive the distal continuum structure to turn along different directions in space; or
wherein the guide driving mechanism is connected with the plurality of second structural backbones, and drives the distal continuum structure to turn along different directions in space by pushing or pulling the second structural backbones.

10. The guidable and growable instrument according to claim 1, wherein the guide comprises a turning member and driving wires disposed within the turning member, wherein distal ends of the driving wires are connected with a distal end of the turning member, and the driving wires are configured to drive the turning member to turn in at least one degree of freedom direction under the drive of a guide driving mechanism.

11. The guidable and growable instrument according to item 10, wherein the turning member is a snake bone structure including a plurality of hollow bamboo-like bending units connected in head-to-tail manner, and through a connecting groove and a connecting protrusion connected with each other, two adjacent bending units form a motion pair that is bendable radially; or
the turning member is a flexible sheath provided with a plurality of slit units along its extension direction at intervals, wherein each of the slit units includes at least one slit extending circumferentially along the flexible sheath; or
the turning member is a bellows, the driving wires can be disposed within and throughout the bellows, or disposed in and throughout a bellows wall of the bellows.

12. The guidable and growable instrument according to claim 1, further comprising a tube driving mechanism connected to the growable tube, for driving the outer layer or the inner layer of the growable tube to move.

13. The guidable and growable instrument according to claim 12, wherein the tube driving mechanism comprises a driving unit, a moving rod, and a transmission unit connected with the driving unit and the moving rod, wherein the moving rod is sealingly connected with the inner layer or the outer layer of the growable tube, and the transmission unit is configured to convert rotary motion of the driving unit into linear motion, so as to drive the moving rod to drive the growable tube to grow or withdraw.

14. The guidable and growable instrument according to claim 13, wherein the transmission unit comprises first and second rollers arranged in parallel;
a proximal end of the moving rod is disposed between the first roller and the second roller and abuts against the first and second rollers, and a distal end of the moving rod is sealingly connected with the inner layer or the outer layer of the growable tube;
the driving unit is connected with the first and second rollers, respectively, for driving the first and second rollers to synchronously rotate in opposite directions at the same speed, so as to drive the moving rod to move linearly.

15. The guidable and growable instrument according to claim 13, wherein the transmission unit comprises a screw slider module including a lead screw and a slider connected by threads;
the proximal end of the moving rod is fixedly connected with the slider, and the distal end of the moving rod is sealingly connected with the inner layer or the outer layer of the growable tube; and
the driving unit is connected with the lead screw for driving the lead screw to rotate, so as to drive the moving rod to move linearly.

16. The guidable and growable instrument according to claim 12, further comprising a fluid controller;
wherein the fluid controller is configured to pressurize or depressurize the fluid, so as to drive the fluid to fill the fluid cavity of the turnable region or drive the fluid to withdraw from the fluid cavity.

17. The guidable and growable instrument according to claim 1, wherein the fluid is a liquid fluid or a gaseous fluid.

18. The guidable and growable instrument according to claim 1, wherein the growable tube is made of flexible material; and/or
the cross-section of the growable tube is circular or oval.

19. The guidable and growable instrument according to claim 1, wherein the guide further comprises a medical instrument fixedly disposed at the distal end of the guide or disposed in an internal channel of the guide;
the medical instrument includes an ultrasound probe, a probe, a drug capsule, or a surgical end effector.

20. A surgical robotic system comprising:
a guidable and growable instrument comprising:
a growable tube comprising an inner layer, an outer layer, and a fluid cavity between the inner layer and the outer layer, the fluid cavity being configured to accommodate a fluid;
wherein the growable tube comprises a turnable region at a distal end, the inner layer and the outer layer are connected and turnable in the turnable region;
a guide arranged in a channel surrounded by the inner layer of the growable tube, wherein a distal end of the guide is bendable to drive the growable tube to turn; and
a system controller configured to control a motion of the guidable and growable instrument.
